# EUROPÄISCHE PATENTSCHRIFT

(11) **EP 3 407 833 B1**
(45) Veröffentlichungstag und Bekanntmachung des Hinweises auf die Patenterteilung: **04.11.2020**
(21) Anmeldenummer: 17705779.1
(22) Anmeldetag: 20.01.2017
(51) Int. Cl.: A61F 2/07, A61F 2/966

(54) **TRANS AORTALER PROXIMALER AORTEN STENT-GRAFT**
TRANS-AORTIC PROXIMAL AORTIC STENT GRAFT
ENDOPROTHÈSE TRANSAORTIQUE POUR AORTE PROXIMALE

(30) Priorität: 25.01.2016 DE 102016101272
(43) Veröffentlichungstag der Anmeldung: 05.12.2018
(73) Patentinhaber: Universität Rostock, 18051 Rostock (DE)
(72) Erfinder: GROSS, Justus, 24235 Laboe (DE)
(74) Vertreter: Hansen, Jochen
(86) Internationale Anmeldenummer: PCT/DE2017/100031
(87) Internationale Veröffentlichungsnummer: WO 2017/129168

(56) Entgegenhaltungen:
- EP-A1- 2 727 563
- WO-A1-03/079935
- WO-A1-2006/037086
- WO-A1-2008/066923
- WO-A1-2015/075708
- WO-A2-2014/108895
- US-A- 5 242 452

## Beschreibung

Die Erfindung betrifft einen Trans Aortalen Proximalen Aorten Stent-Graft [TAPAS-Graft] zum Einführen in ein Blutgefäß, mit einer schlauchartigen Schleuse, die eine gefaltete, selbstexpandierende Prothese sowohl von innen, als auch von außen derart umhüllt, dass eine Schleusenaußenwand der Schleuse von außen an der Prothese anliegt und eine Schleuseninnenwand von innen an der Prothese anliegt und die Prothese gefaltet hält, wobei an einem distalen Ende der Prothese diese von der Schleuse in einem Übergang von der Schleuseninnenwand in die Schleusenaußenwand umstülpt ist und die Prothese an einem proximalen Ende mit einer Verankerung versehen ist.

Aus dem **Stand der Technik** ist beispielsweise aus der Druckschrift DE 10 2012 101 103 B3 ein Stentgraft mit Fixierelementen und Einführsystem bekannt. Der Stentgraft weist dabei Abschnitte mit einem selbstexpandierenden Stent aus, die in ihrer Längsrichtung hintereinander angeordnete Ringe aus mäanderförmigen Stützen mit einem an Ringen befestigten Prothesenmaterial auf. Diese Ringe weisen jeweils Fixierbereiche mit schlaufenförmigen Fixierelementen auf.

Ferner zeigt die Druckschrift DE 60 2005 005 567 T2 eine Einführungsvorrichtung mit einer niedrigen Entfaltungskraft, die eine schrittweise Entfaltung definierte Stentabschnitte ermöglicht.

Die Druckschrift WO 2014/108895 A2 beschreibt ein Stentgraft System, bei dem ein Mechanismus zur Minimalisierung der Stent-Graft Migration während der Applikation im Vordergrund steht, wobei die Entfaltung von der Device-Spitze her von distal nach proximal erfolgt.

Aus der Druckschrift WO 2015/075708 A1 ist ein Stentgraft System bekannt, wobei ein Mechanismus mit einer radiären Expansionskontrolle während der Applikation dargestellt wird, wobei die Entfaltung von der Device-Spitze her von distal nach proximal erfolgt.

Weiter wird in der Druckschrift EP 2 727 563 A1 ein Stentgraft System mit einem Mechanismus beschrieben, bei dem eine gesonderte zeitverzögerte Entfaltung der Stentgraft-Spitze während der Applikation, die von der Device-Spitze her von distal nach proximal erfolgt, ermöglicht ist.

Die Druckschrift WO 2008/066923 A1 beschreibt einen Mechanismus zum Auslösen eines Stentgrafts mittels Seilzug, bei dem die Entfaltung ebenfalls von der Device-Spitze her von distal nach proximal erfolgt.

Ferner beschreibt die DruckschriftWO 2006/037086 A1 eine Kombination zweier Stentgraft-Systeme, wobei ein flexibler gecoverter Anteil des ersten Stentgrafts zur proximalen Fixierung der B-Dissektion genutzt wird, ein zweites ungecovertes Stentgraft-System an das erste angedockt wird und eine langstreckige Fixation der dissektionsbedingten freien Intima gewährleistet werden soll, wobei die Entfaltung von der Device-Spitze her von distal nach proximal erfolgt.

Die Druckschrift US 5,242,452 B1 beschreibt einenMechanismus zum Auslösen eines Stentgrafts mittels Seilzug, bei dem die Entfaltung von der Device-Spitze her von distal nach proximal erfolgt.

Weiter ist aus der Druckschrift WO 03/079935 A1 ein Mechanismus zum Auslösen eines Stentgrafts mittels Seilzug bekannt, wobei die Entfaltung ebenfalls von der Device-Spitze her von distal nach proximal erfolgt.

EP 2554141 (A1) zeigt eine Einführschleuse mit einerröhrenförmige Struktur, die auf einen gewünschten Durchmesser dehnbar und zusammenziehbar ist. Ein Aktuator umfasst ein inneres Element und ein äußeres Element. In Reaktion auf eine Relativbewegung zwischen den Elementen und der Rohrstruktur in einer ersten oder zweiten Richtung ermöglicht der Aktuator, dass sich die Rohrstruktur zwischen der ersten oder zweiten Konfiguration bewegt.

Der vorliegenden Erfindung liegt die **Aufgabe** zugrunde, einen Trans Aortalen Proximalen Aorten Stent-Graft (TAPAS-Graft) bereit zu stellen, der antegrad kontrolliert minimalinvasiv in ein Blutgefäß einbringbar ist und eine gefaltete Prothese einsetzt und vom proximal Ende nach dem distalen Ende der Prothese hin entfaltet.

Eine weitere Aufgabe ist es eine Prothese des erfinderischen Trans Aortalen Proximalen Aorten Stent-Graft (TAPAS-Graft) exakt in einem Körper zu positionieren und einzusetzen.

Eine weitere Aufgabe ist es die eingesetzte Prothese des Trans Aortalen Proximalen Aorten Stent-Graft (TAPAS-Graft) nach Einsetzen durch eine Führung von dieser Führung durch einen Auslösemechanismus von der Prothese zu trennen und zusammen mit einer Schleuse aus dem Blutgefäß herauszuziehen.

Diese Aufgaben werden durch den erfinderischen Trans Aortaler Proximaler Aorten Stent-Graft **gelöst.**

Der Trans Aortale Proximale Aorten Stent-Graft [TAPAS-Graft] zum Einführen in ein Blutgefäß, ist mit einer schlauchartigen Schleuse ausgebildet, die eine gefaltete, selbstexpandierende Prothese sowohl von innen, als auch von außen derart umhüllt, so dass eine Schleusenaußenwand der Schleuse von außen an der Prothese anliegt und eine Schleuseninnenwand von innen an der Prothese anliegt und die Prothese gefaltet hält, wobei an einem distalen Ende der Prothese diese von der Schleuse in einem Übergang von der Schleuseninnenwand in die Schleusenaußenwand umstülpt ist und die Prothese an einem proximalen Ende mit einer Verankerung versehen ist.

Ferner ist in die Schleuse eine Führung, insbesondere ein Führungskatheter, einbringbar, der mit der Verankerung an der Prothese lösbar über eine lösbare Fixierung mit Auslösemechanismus verbindbar ist.

Die lösbare Fixierung ist mit Auslösemechanismus als ein Kordel-Splint-Mechanismus ausgeführt.

Das Verfahren zum Einsatz eines hier offenbarten und beschriebenen Trans Aortalen Proximalen Aorten Stent-Graft [TAPAS-Graft], weist die Schritte auf:
- Fixierung des TAPAS-Graft an der Führung, indem die lösbare Fixierung mit Auslösemechanismus die Führung mit der Verankerung an der Prothese verbindet,
- Einbringen des TAPAS-Graft über die Führung (in ein Blutgefäß), bevorzugt unter Bildwandler (BV-) Kontrolle,
- Freisetzen des TAPAS-Graft, indem durch ein Herausziehen der Schleuse die gefaltete, selbstexpandierende Prothese vollständig freigegeben und entfaltet wird und damit die Prothese an einer Blutgefäßwand zur Anlage kommt,
- Positionieren der Prothese über die mit der Verankerung an der Prothese und lösbare Fixierung mit Auslösemechanismus an der Verankerung fixierte Führung,
- Trennung der Prothese von der Führung durch Betätigung des Auslösemechanismus,
- Entfernen der Führung mit der Schleuse.

Die innovative Stent-Graftprothese ist unter Gebrauch eines antegraden operativen Zugangs geeignet zur Behandlung von:
a) B-Dissektion
b) Intramurales Hämatome der Aorta descendens
c) Penetrierendes Aortenulcus
d) Aneurysma (verum oder dissekans der Aorta descendens)
   (a-d) bei ausgeprägter Verkalkung bzw. Verschluss der abdominellen Aorta, der Becken- und Leistenarterien
e) A-Dissektion
   - Nach Ascendens- oder (Teil-) Bogenersatz erfolgt transprothesial die Stentbehandlung der residuellen Dissektion in der proximalen Aorta descendens
   - Verzicht auf den Einsatz eines "Elephant-Trunks" bzw. "frozen Elephant Trunks".

Zur Funktionsweise des TAPAS- Graft:
Der chirurgische Zugang kann
a) transaortal über den Aortenbogen
b) über die linke A. subclavia
c) über eine eingebrachte Ascendensprothese erfolgen.

Baulich basiert der TAPAS-Graft auf der Möglichkeit, die Stent-Graftprothese unter BV-Kontrolle antegrad einzubringen und von proximal nach distal auslösen zu können. Damit ist eine exakte Platzierung des proximalen Anteils gewährleistet.

Der Stentgraft wird zwischen der Schleuseninnenwand und der Schleusenaußenwand "gepackt" angeordnet und über einen steifen Führungskatheter, zum Beispiel Back up Meyer oder Lundaquist unter BV-Kontrolle in das Aortenlumen geführt. Die Prothese ist über einen bereits offenbarten Platzierungs- und einen Haltemechanismus über einen Kordel-Splint-Mechanismus an das proximale Trägersystem gekoppelt.

Die Schleuse des TAPAS-Grafts weist eine verkürzte äußere und eine durchgehende innere Wand auf, wobei die beiden Schichten am distalen Ende miteinander verbunden sind. Durch manuelles Vorschieben der Schleuse erfolgt ein successives Entfalten des Stentgrafts, wenn das Ende der äußeren Schleusenwand an dem "gepackten" Stentgraft vorbeigeschoben wird.

Nach dem kompletten Entfalten des Stentgrafts kann die Prothese über den Kordel-Splint-Mechanismus positioniert und freigesetzt werden. Die Entfernung der gesamten Schleuse erfolgt dann retrograd durch das Lumen des Endostentgrafts.

Der Aufbau des Trans Aortalen Proximalen Aorten Stent-Grafts (TAPAS-Graft) ermöglicht eine kompakte Bereitstellung eines Stent-Graft-Systems mit schlauchartig, gefalteter Prothese, die durch eine Führung, beispielsweise einen Führungskatheter, der mit einem Auslösemechanismus versehbar ist an einer Prothese lösbar, fixierbar ist.

Nachfolgend werden Ausführungsbeispiele der Erfindung anhand der beiliegenden Zeichnungen in der **Figurenbeschreibung** detailliert beschrieben, wobei diese die Erfindung erläutern sollen und nicht beschränkend zu werten sind:
Es zeigen:
- Fig. 1: eine erfinderische Vorrichtung in erster Position im Querschnitt;
- Fig. 2: eine erfinderische Vorrichtung in erster Position gemäß Fig. 1 im Längsschnitt;
- Fig. 3: eine proximale Verankerung an einer Prothese in erster Position im Querschnitt;
- Fig. 4: eine proximale Verankerung des Auslösemechanismus in erster Position in perspektivischer seitlicher Ansicht und
- Fig. 5: eine erfinderische Vorrichtung in zweiter Position im Längsschnitt.

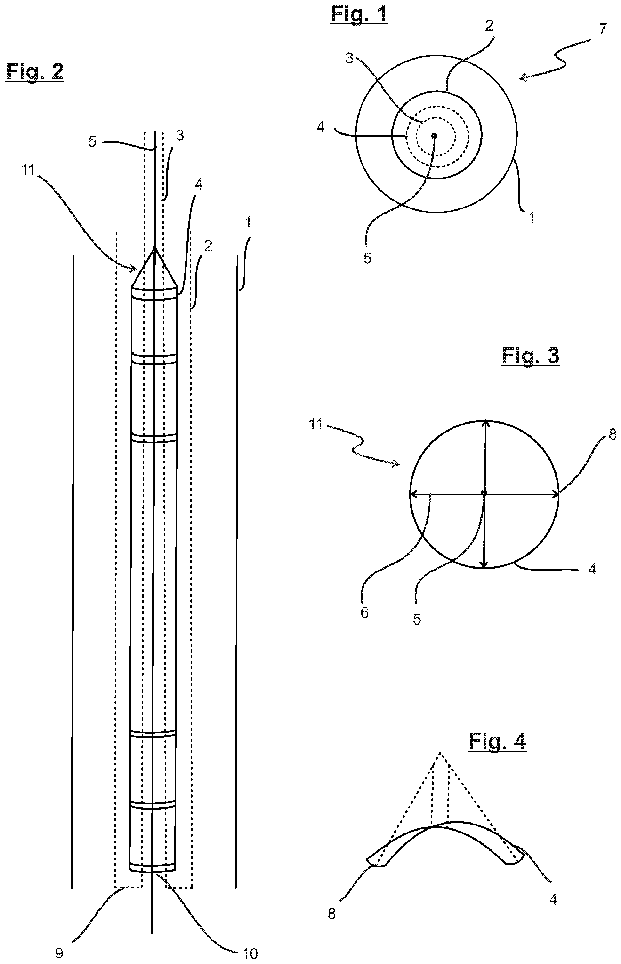

In **Fig. 1** ist ein erfinderischer TAPAS-Graft 7 in einer ersten Position im Querschnitt dargestellt. Von außen nach innen zeigt die Figur eine Blutgefäßwand 1 und ein zwischen einer Schleusenaußenwand 2 und Schleuseninnenwand 3 gehaltene schlauchartige, gefaltete Prothese 4.

**Fig. 2** zeigt einen erfinderischen TAPAS-Graft 7 in einer ersten Position im Längsschnitt. Von außen nach innen zeigt die Figur eine Blutgefäßwand 1 und eine zwischen einer Schleusenaußenwand 2 und Schleuseninnenwand 3 gehaltene Prothese 4. Der erfinderische TAPAS-Graft 7 ist dabei an einer mittig befindlichen Führung 5, insbesondere einem

Führungskatheter oder auch Guide Wire, über eine Verankerung 8 an der Prothese 4 lösbar fixiert. Die Verankerung 8 an der Prothese 4 ist mit einer lösbaren Fixierung mit einem Auslösemechanismus 6 an der Führung 5 mit dem, in dieser Figur oben dargestellten, proximalen Ende der Prothese 4 verbunden, wobei dieser, vorzugsweise durch einen Kordel-Splint-Mechanismus, derart wirkt, dass nach einer Entfaltung der Prothese 4 mit einem Herausziehen der Schleuse und der Führung die Verbindung der Prothese 4 mit der Führung 5 durch den Auslösemechanismus 6 gelöst werden kann.

**Fig. 3** zeigt eine Verankerung 8 mit lösbarer Fixierung 6 an der Führung 5 des Auslösemechanismus in erster Position im Querschnitt am proximalen Ende 11der Prothese 4. Hier kann z.B. ein Kordel-Splint-Mechanismus wirken.

**Fig. 4** zeigt eine Verankerung 8 mit lösbarer Fixierung 6 des Auslösemechanismus in erster Position im Querschnitt am proximalen Ende 11 der Prothese 4.

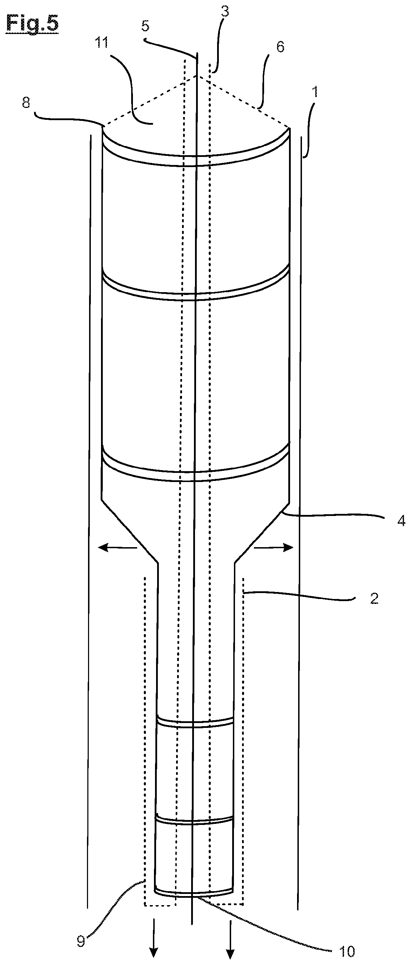

**Figur 5** zeigt einen erfinderischen TAPAS-Graft 7 in einer zweiten Position im Längsschnitt. Diese zweite Position zeigt eine partielle Entfaltung der Prothese 4, durch eine bereichsweise Wegnahme bzw. Verschiebung der Schleusenaußenwand 2, wobei sich einzelne Elemente/Abschnitte der Prothese 4 im oberen Bereich der Figur an die Blutgefäßwand 1 anschmiegen. Die Schleuse 9 wird durch ein Herausziehen an der Führung 5 bewegt und setzt damit den Entfaltungsprozess der Prothese 4 frei.

Im unteren Teil der Figur, also an distalen Ende 10 ist die Prothese 4 noch zwischen der Schleusenaußenwand 2 und Schleuseninnenwand 3 gehalten.

Der erfinderische TAPAS-Graft 4 ist dabei an einer mittig befindlichen Führung 5 lösbar fixiert. Die lösbare Fixierung 6 erfolgt an dem (in der Figur oben) gezeigten proximalen Ende der Prothese 4 und wird vorzugsweise erst nach der voll-ständigen Entfaltung der Prothese 4 von dieser gelöst und mit der Führung 5 herausgezogen.

### Bezugszeichenliste

- 1: Blutgefäßwand
- 2: Schleusenaußenwand
- 3: Schleuseninnenwand
- 4: Prothese
- 5: Führung
- 6: lösbare Fixierung mit Auslösemechanismus
- 7: TAPAS-Graft
- 8: Verankerung
- 9: Schleuse
- 10: distales Ende
- 11: proximales Ende

## Patentansprüche

1. Trans Aortaler Proximaler Aorten Stent-Graft [TAPAS-Graft] (7) zum antegraden Einführen in ein Blutgefäß, mit einer schlauchartigen Schleuse, die eine gefaltete, selbstexpandierende Prothese (4) sowohl von innen, als auch von außen derart umhüllt, dass eine Schleusenaußenwand (2) der Schleuse von außen an der Prothese (4) anliegt und eine Schleuseninnenwand (3) von innen an der Prothese anliegt und die Prothese (4) gefaltet hält,
wobei
an einem distalen Ende der Prothese (4) diese von der Schleuse in einem Übergang von der Schleuseninnenwand (3) in die Schleusenaußenwand (4) umstülpt ist und die Prothese (4) an einem proximalen Ende mit einer Verankerung (8) versehen ist,
und wobei
in die Schleuse eine Führung (5), insbesondere ein Führungskatheter, einbringbar ist, der mit der Verankerung (8) an der Prothese (4) lösbar über eine lösbare Fixierung mit Auslösemechanismus (6) verbindbar ist,
**dadurch gekennzeichnet, dass**
die lösbare Fixierung mit Auslösemechanismus (6) als ein Kordel-Splint-Mechanismus ausgeführt ist
und
der Auslösemechanismus so positioniert ist, dass die Prothese von proximal nach distal auslösbar ist.

## Claims

1. A trans-aortic proximal aortic stent graft [TAPAS graft] (7) for antegrade introduction into a blood vessel, having a hose-like sheath which surrounds a folded, self-expanding prosthesis (4) both internally and externally in such a manner that a sheath outer wall (2) of the sheath rests against the prosthesis (4) externally and a sheath inner wall (3) rests against the prosthesis internally and keeps the prosthesis (4) folded,
wherein
at a distal end of the prosthesis (4), the latter is everted by the sheath in a transition from the sheath inner wall (3) into the sheath outer wall (2) and the prosthesis (4) is provided with an anchoring means (8) at a proximal end,
and wherein
a guide (5), in particular a guide catheter, is insertable into the sheath, which guide catheter is connectable to the anchorage (8) at the prosthesis in a detachable manner via a detachable fixing means with release mechanism (6),
**characterised in that**
the detachable fixing means with release mechanism (6) is in the form of a cord-splint mechanism
and
the release mechanism is positioned in such a manner that the prosthesis is releasable from proximal to distal.

## Revendications

1. Endoprothèse transaortique proximale pour aorte [TAPAS-Graft] (7) à insérer de façon antérograde dans un vaisseau sanguin, comprenant une gaine tubulaire qui entoure à la fois de l'intérieur et de l'extérieur une prothèse comprimée auto-expansible (4) de telle sorte qu'une paroi extérieure de gaine (2) de la gaine repose à l'extérieur sur la prothèse (4) et une paroi intérieure de gaine (3) de la gaine repose à l'intérieur sur la prothèse, et maintient la prothèse (4) comprimée,
la prothèse (4) étant, à une extrémité distale de la prothèse (4), retournée par la gaine dans une transition de la paroi intérieure de gaine (3) à la paroi extérieure de gaine (2), et la prothèse (4) étant munie d'un ancrage (8) à une extrémité proximale, et
un guide (5), en particulier un cathéter de guidage, pouvant être introduit dans la gaine, lequel guide peut être relié de manière réversible à la prothèse (4) avec l'ancrage (8) par l'intermédiaire d'une fixation réversible à mécanisme de déclenchement (6),
**caractérisé en ce que**
la fixation réversible à mécanisme de déclenchement (6) est conçue comme un mécanisme de cordon-goupille et
le mécanisme de déclenchement est positionné de telle sorte que la prothèse peut être déployée de l'extrémité proximale à l'extrémité distale.
